# EUROPEAN PATENT APPLICATION

(11) **EP 4 699 606 A2**
(43) Date of publication of application: **25.02.2026**
(21) Application number: 25217941.1
(22) Date of filing: 04.10.2022
(51) Int. Cl.: A61K 31/438

(54) **MODULATORS OF THE BETA-3 ADRENERGIC RECEPTOR USEFUL FOR THE TREATMENT OR PREVENTION OF DISORDERS RELATED THERETO**

(30) Priority: 06.10.2021 US 202163253060 P
(62) Divisional of application: 22786520.1
(71) Applicant: Arena Pharmaceuticals, Inc., New York, NY 10001-2192 (US)
(72) Inventor: BLACKBURN, Anthony C., New York, NY 10001-2192 (US)
(74) Representative: Pfizer

(57) **Abstract**

Provided is a solvate that is 1-ethyl-3-((R)-3-((S)-2-hydroxy-3-(3-(methylsulfonyl)phenoxy)propylamino)-1-oxa-8-azaspiro[4.5]decan-8-ylsulfonyl)quinolin-4(1H)-one mesylate hemihydrate Form 2, and pharmaceutical compositions thereof that modulate the activity of the beta-3 adrenergic receptor.

## Description

Heart failure (HF) is a clinical syndrome characterized by symptoms (e.g., breathlessness, ankle swelling, and fatigue) that may be accompanied by signs (e.g., elevated jugular venous pressure, pulmonary crackles, and peripheral edema) caused by structural and/or functional cardiac abnormalities. HF can result from a number of underlying conditions that damage, weaken, or stiffen the heart leading to compromised cardiac function, including coronary artery disease, myocardial infarction, hypertension, defects in cardiac valves, cardiomyopathies, myocarditis, arrhythmias, or other congenital and chronic conditions.

Acute heart failure is a rapid decline in heart function that can cause anoxia of tissues (particularly the brain), leading to death. Acute heart failure can occur in previously asymptomatic individuals (e.g., individuals with pulmonary edema or cardiogenic shock), or in individuals with an acute exacerbation of chronic heart failure.

In the healthy heart, the actions of beta-1 and beta-2 adrenergic receptors are dominant and act through a Gs-coupled pathway to increase the force and frequency of myocardial contraction, while beta-3 adrenergic receptors act through a Gi-coupled eNOS pathway to exert weak negative inotropic effects. In the failing heart, beta-1 and beta-2 adrenergic receptors are downregulated or desensitized, while beta-3 adrenergic receptors are upregulated, thereby emphasizing the negative effects of beta-3 agonism on cardiac contractility.

In individuals experiencing acute heart failure, the short-term goal is to increase contractility and improve hemodynamic status. The current standard of care for acute heart failure includes the administration of inotropes-agents that alter the force or energy of cardiac contractions. These agents are typically administered in an intensive care setting by continuous injection. Examples of such agents include adrenaline, dobutamine, dopamine, levosimendan, and noradrenaline. However, the initial improvement in contractility afforded by these agents can be followed by accelerated mortality. The excessive mortality following administration of these agents has been linked to increased tachycardia and myocardial oxygen consumption that leads to arrhythmia and myocardial ischemia.

### SUMMARY

Provided is a solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 2 (Compound A).

Also provided are processes for making a solvate that is Compound A and products of those processes.

Also provided is a solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 1 (Compound B).

Also provided is amorphous 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate.

Also provided is a composition comprising a compound described herein.

Also provided is a pharmaceutical composition comprising a compound described herein and a pharmaceutically acceptable carrier.

Also provided is a dosage form comprising a compound described herein and a pharmaceutically acceptable carrier.

Also provided is a method for treating or preventing a beta-3 adrenergic receptor-mediated disorder in an individual, comprising administering to said individual in need thereof, a therapeutically effective amount of a compound described herein or a pharmaceutical composition or a dosage form thereof.

These and other aspects of the invention disclosed herein will be set forth in greater detail as the patent disclosure proceeds.

### BRIEF DESCRIPTION OF THE DRAWINGS

**FIG. 1 (Figure 1****)** shows a powder X-ray diffraction (PXRD) pattern for a sample containing the solvate that is Compound A.
**FIG. 2 (Figure 2****)** shows a thermogravimetric analysis (TGA) thermogram of a sample containing the solvate that is Compound A.
**FIG. 3 (Figure 3****)** shows a differential scanning calorimetry (DSC) thermogram for a sample containing the solvate that is Compound A.
**FIG. 4A** **and** **FIG.4B** **(****Figure 4A** **and** **Figure 4B****)** show a dynamic moisture sorption (DMS) profile of a sample containing the solvate that is Compound A.
**FIG. 5 (Figure 5****)** shows a powder X-ray diffraction (PXRD) patterns for a sample containing the solvate that is Compound A (bottom) and the solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form I (Compound B, top).
**FIG. 6 (Figure 6****)** shows images of a crystalline batch of Compound A (left) and the single crystal selected for data collection (right).

### DETAILED DESCRIPTION OF THE INVENTION

### DEFINITIONS

For clarity and consistency, the following definitions will be used throughout this patent document.

As used herein, "**Compound A**" refers to 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 2.

As used herein, "**Compound B**" refers to 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 1.

As used herein, "**Compound C**" refers to 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate, including amorphous and crystalline forms thereof, including but not limited to solvates, such as hydrates.

As used herein, "**Compound D**" refers to 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one, i.e., the free base of Compound C, including amorphous and crystalline forms thereof, including but not limited to solvates, such as hydrates.

As used herein, "**administering**" refers to providing a compound of the invention or other therapy, remedy or treatment to the individual in need of treatment in a form that can be introduced into that individual's body in a therapeutically useful form and therapeutically useful amount, including, but not limited to: oral dosage forms, such as tablets, capsules, syrups, suspensions, and the like; injectable dosage forms, such as IV, IM, or IP, and the like; transdermal dosage forms, including creams, jellies, powders, or patches; buccal dosage forms; inhalation powders, sprays, suspensions, and the like; and rectal suppositories. A health care practitioner can directly provide a compound to an individual in the form of a sample, or can indirectly provide a compound to an individual by providing an oral or written prescription for the compound. Also, for example, an individual can obtain a compound by themselves without the involvement of a health care practitioner. When the compound is administered to the individual, the body is transformed by the compound in some way. When a compound of the invention is provided in combination with one or more other agents, "administration" is understood to include the compound and other agents are administered at the same time or at different times. When the agents of a combination are administered at the same time, they can be administered together in a single composition or they can be administered separately.

The term "**antagonist**" as used herein refers to a moiety that can competitively bind to the β₃-adrenergic receptor as an agonist (for example, the endogenous ligand) but does not activate or substantially reduces the intracellular response compared to an agonist, and can thereby inhibit the intracellular responses by an agonist or partial agonist. An "antagonist" does not diminish the baseline intracellular response, or does so to a negligible extent, in the absence of an agonist or partial agonist.

The phrase "**as depicted in**" with reference to a Figure refers to the crystal form and/or morphology as being characterized by graphical data "as depicted in" the Figure. Such data include, for example, powder X-ray diffractograms, differential scanning calorimetry traces, and dynamic moisture sorption graphs. The skilled person will understand that such graphical representations of data may be subject to small variations, *e.g*., in peak relative intensities and peak positions due to factors such as variations in instrument response and variations in sample concentration and purity, which are well known to the skilled person. Nonetheless, the skilled person would readily be capable of comparing the graphical data in the Figures herein with graphical data generated for an unknown crystal form and/or morphology (habit) and confirm whether the two sets of graphical data are characterizing the same crystal form (or morphology) or two different crystal forms (or morphologies). A crystalline free-plate habit of the Compound A referred to herein as being characterized by graphical data "as depicted in" a Figure will thus be understood to include any morphologies characterized with the graphical data having such small variations, as are well known to the skilled person, in comparison with the Figure.

The term "**composition**" refers to a compound described herein, in combination with at least one additional component, such as, a composition obtained/prepared during synthesis, preformulation, in-process testing (*i.e*., TLC, HPLC, NMR samples), and the like.

The term "**hydrate**" as used herein means a compound that further includes a stoichiometric or non-stoichiometric amount of water bound by non-covalent intermolecular forces.

The term "**in need of treatment**" and the term "**in need thereof**" when referring to treatment are used interchangeably to mean a judgment made by a caregiver (*e.g*. physician, nurse, nurse practitioner, *etc.* in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual or animal is ill, or will become ill, as the result of a disease, condition or disorder that is treatable by the compounds of the invention. Accordingly, the compounds of the invention can be used in a protective or preventive manner; or compounds of the invention can be used to alleviate, inhibit, or ameliorate the disease, condition, or disorder.

The term "**individual**" refers to any animal, including mammals, such as, mice, rats, other rodents, rabbits, dogs, cats, swine, cattle, sheep, horses, primates, and humans. In some embodiment "individual" refers to humans.

The term "**pharmaceutical composition**" refers to a specific composition comprising at least one active ingredient whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

The term "**prescribing**" refers to order, authorize, or recommend the use of a drug or other therapy, remedy, or treatment. In some embodiments, a health care provider orally advises, recommends, or authorizes the use of a compound, dosage regimen, or other treatment to an individual. The health care provider may or may not provide a written prescription for the compound, dosage regimen, or treatment. Further, the health care provider may or may not provide the compound or treatment to the individual. For example, the health care provider can advise the individual where to obtain the compound without providing the compound. In some embodiments, a health care provider can provide a written prescription for the compound, dosage regimen, or treatment to the individual. A prescription can be written on paper or recorded on electronic media. In addition, a prescription can be called in (oral) or faxed in (written) to a pharmacy or a dispensary. In some embodiments, a sample of the compound or treatment is given to the individual. As used herein, giving a sample of a compound constitutes an implicit prescription for the compound. Different health care systems around the world use different methods for prescribing and administering compounds or treatments, and these methods are encompassed by the disclosure herein.

A health care provider can include, for example, a physician, nurse, nurse practitioner, or other health care professional who can prescribe or administer compounds (drugs) for the disorders disclosed herein. In addition, a health care provider can include anyone who can recommend, prescribe, administer, or prevent an individual from receiving a compound or drug, including, for example, an insurance provider.

The terms "**prevent**," "**preventing**," and "**prevention**" refer to the elimination or reduction of the occurrence or onset of one or more symptoms associated with a particular disorder. For example, the terms "prevent," "preventing," and "prevention" can refer to the administration of therapy on a prophylactic or preventative basis to an individual who may ultimately manifest at least one symptom of a disorder but who has not yet done so. Such individuals can be identified on the basis of risk factors that are known to correlate with the subsequent occurrence of the disease, such as the presence of a biomarker. Alternatively, prevention therapy can be administered as a prophylactic measure without prior identification of a risk factor. Delaying the onset of the at least one episode and/or symptom of a disorder can also be considered prevention or prophylaxis.

The term "**solvate**" as used herein means a compound that further includes a stoichiometric or non-stoichiometric amount of a solvent bound by non-covalent intermolecular forces. Preferred solvents are volatile, non-toxic, and/or acceptable for administration to humans in trace amounts.

The terms "**treat**," "**treating**," and "**treatment**" refer to the administration of therapy to an individual who already manifests, or who has previously manifested, at least one symptom of a disease, disorder, condition, dependence, or behavior. For example, "treating" can include any of the following with respect to a disease, disorder, condition, dependence, or behavior: alleviating, abating, ameliorating, improving, inhibiting (*e.g*., arresting the development), relieving, or causing regression. "Treating" can also include treating the symptoms, preventing additional symptoms, preventing the underlying physiological causes of the symptoms, or stopping the symptoms (either prophylactically and/or therapeutically) of a disease, disorder, condition, dependence, or behavior. For example, the term "treating" in reference to a disorder means a reduction in severity of one or more symptoms associated with a particular disorder. Therefore, treating a disorder does not necessarily mean a reduction in severity of all symptoms associated with a disorder and does not necessarily mean a complete reduction in the severity of one or more symptoms associated with a disorder.

The term "**therapeutically effective amount**" refers to the amount of active compound or pharmaceutical agent that elicits the biological or medicinal response in a tissue, system, animal, or human that is being sought by an individual, researcher, veterinarian, medical doctor, or other clinician or caregiver, which can include one or more of the following:
(1) preventing the disorder, for example, preventing a disease, condition, or disorder in an individual who may be predisposed to the disease, condition, or disorder but does not yet experience or display the relevant pathology or symptomatology;
(2) inhibiting the disorder, for example, inhibiting a disease, condition, or disorder in an individual who is experiencing or displaying the relevant pathology or symptomatology (*i.e*., arresting further development of the pathology and/or symptomatology); and
(3) ameliorating the disorder, for example, ameliorating a disease, condition, or disorder in an individual who is experiencing or displaying the relevant pathology or symptomatology (*i.e*., reversing the pathology and/or symptomatology).

### COMPOUNDS

Provided is a solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 2 (Compound A).

In some embodiments, the solvate that is Compound A exhibits a change in weight of about 0.3% by dynamic moisture-sorption analysis at 25°C and ~0 to 90% relative humidity.

In some embodiments, the solvate that is Compound A displays an X-ray powder diffraction pattern comprising peaks, in terms of 2*θ*, at 15.3° ± 0.2°, 19.1° ± 0.2°, 17.8° ± 0.2°, and 18.9° ± 0.2°.

In some embodiments, the solvate that is Compound A displays an X-ray powder diffraction pattern comprising peaks, in terms of 2*θ*, at 15.3° ± 0.2°, 19.1° ± 0.2°, 17.8° ± 0.2°, 18.9° ± 0.2°, 26.6° ± 0.2°, 22.9° ± 0.2°, and 8.8° ± 0.2°.

In some embodiments, the solvate that is Compound A displays an X-ray powder diffraction pattern comprising peaks, in terms of 2*θ*, at 15.3° ± 0.2°, 19.1° ± 0.2°, 17.794° ± 0.2°, 18.9° ± 0.2°, 26.6° ± 0.2°, 22.9° ± 0.2°, 8.8° ± 0.2°, 12.6° ± 0.2°, 11.4° ± 0.2°, 18.3° ± 0.2°, and 19.9° ± 0.2°.

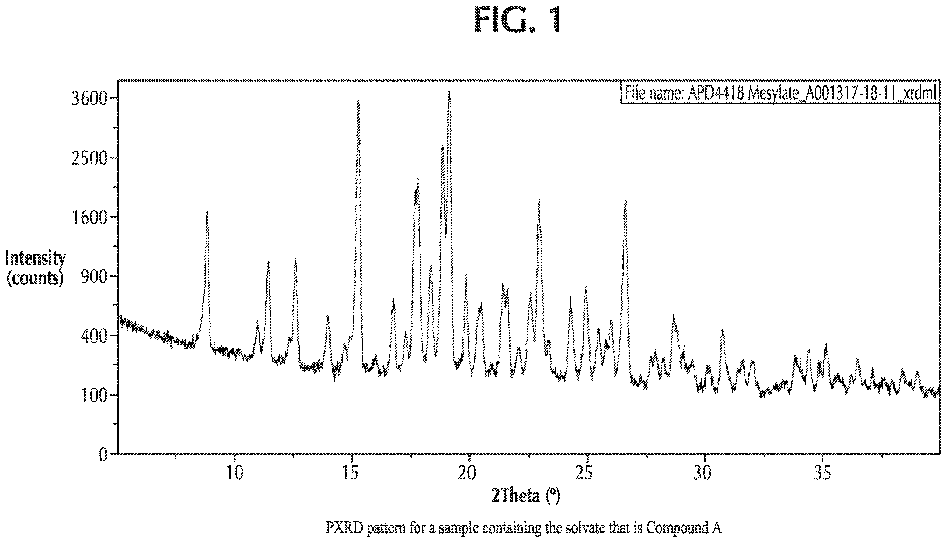

In some embodiments, the solvate that is Compound A displays a powder X-ray diffraction pattern substantially as depicted in Figure 1 wherein substantially means that the reported peaks can vary, in terms of 2*θ*, by about ± 0.2°.

In some embodiments, the solvate that is Compound A the solvate displays a differential scanning calorimetry thermogram comprising an endotherm with an extrapolated onset temperature between about 168 °C and about 176°C.

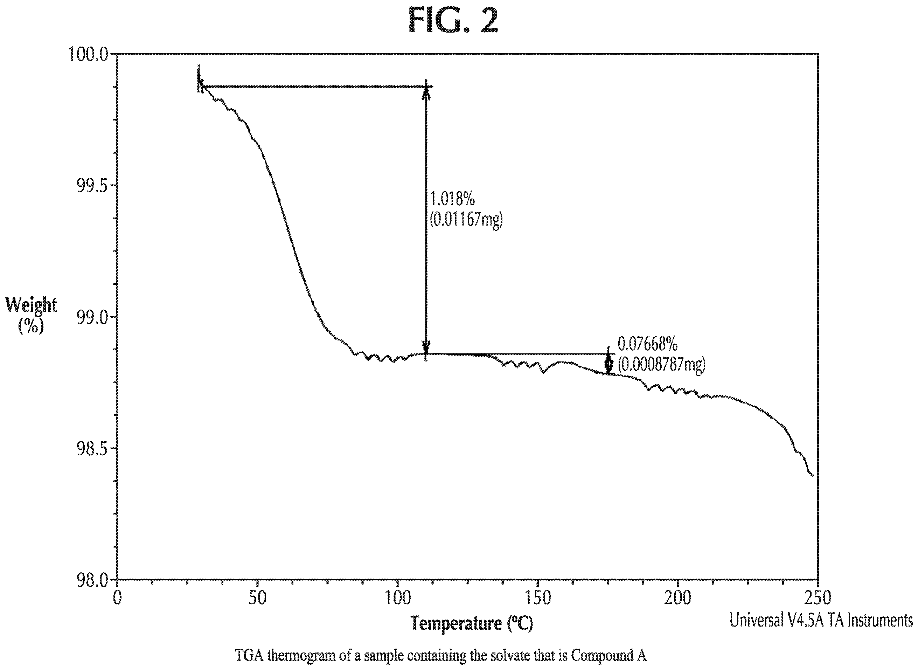

In some embodiments, the solvate that is Compound A has a differential scanning calorimetry thermogram substantially as depicted in Figure 2, wherein substantially means that the reported DSC features can vary by about ± 4 °C and that the reported DSC features can vary by about ± 20 joules per gram.

In some embodiments, the solvate that is Compound A displays a thermogravimetric analysis profile showing about 0.97% weight loss prior to melt.

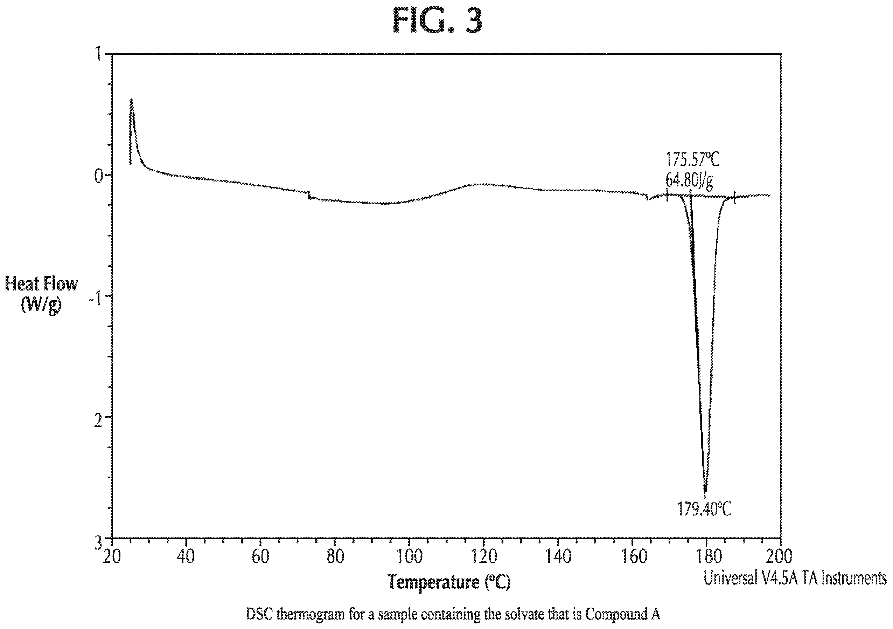

In some embodiments, the solvate that is Compound A the solvate displays a thermogravimetric analysis profile substantially as depicted in Figure 3 wherein substantially means that the reported TGA features can vary by about ± 5 degrees °C and that the reported TGA features can vary by about ± 2% weight change.

In some embodiments, the solvate that is Compound A displays an aqueous solubility of about 28 mgA/mL.

In some embodiments, the solvate that is Compound A is characterized by a triclinic crystal system, as determined by single crystal X-ray analysis.

In some embodiments, the solvate that is Compound A is characterized by a P1 space group, as determined by single crystal X-ray analysis.

In some embodiments, the solvate that is Compound A is characterized by a unit cell, as determined by single crystal X-ray analysis, of the following dimensions: *a* = 8.7267(2) Å, *b* = 10.6750(3) Å, *c* = 18.3096(5) Å, α= 75.811, β= 89.605, χ = 80.968.

In some embodiments, the solvate that is Compound A is characterized by a colourless cut block habit.

Also provided is a solvate that is Compound A prepared by any of the processes described herein.

Also provided is a solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 1 (Compound B).

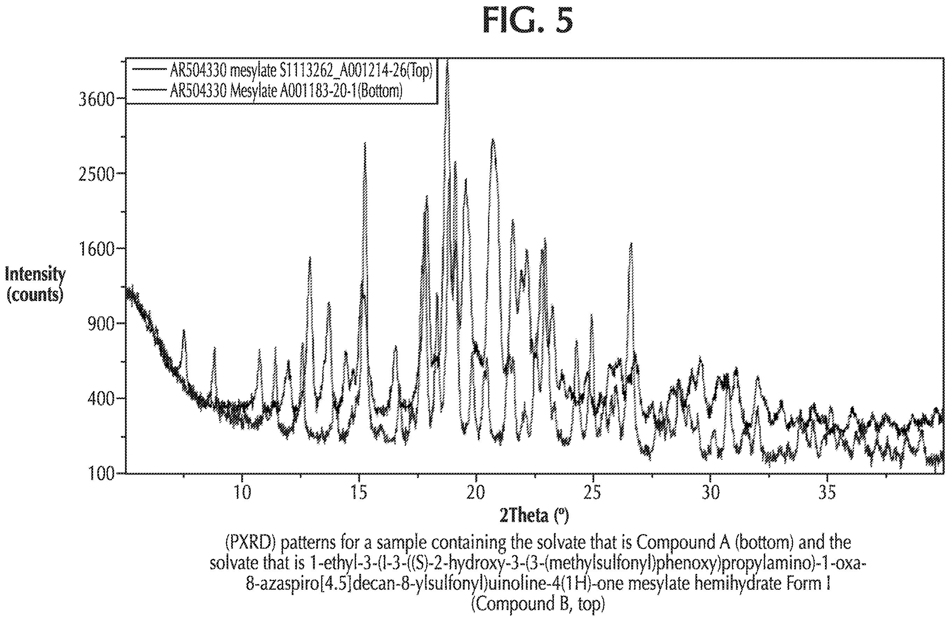

In some embodiments, the solvate that is Compound B displays a powder X-ray diffraction pattern substantially as depicted in Figure 5 wherein substantially means that the reported peaks can vary, in terms of 2*θ*, by about ± 0.2°.

In some embodiments, the solvate that is Compound B the solvate displays a differential scanning calorimetry thermogram comprising an endotherm with an extrapolated onset temperature between about 203 °C and about 206°C.

In some embodiments, the solvate that is Compound B displays a thermogravimetric analysis profile showing about 0.75% weight loss prior to melt.

Also provided is amorphous 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate.

### PROCESSES

Provided is a process for making a solvate that is Compound A comprising the steps of: contacting 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate with acetonitrile, and isolating the solvate that is Compound A.

Also provided is a process for making a solvate that is Compound A comprising the steps of: contacting 1-ethyl-3-((*R*)-3-((S)-2-hydroxy-3-(3-(methylsulfonyl)phenoxy)propylamino)-1-oxa-8-azaspiro[4.5]decan-8-ylsulfonyl)quinolin-4(1*H*)-one mesylate Form 1 with water, and isolating the solvate that is Compound A.

The compound that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate (Compound C) can be prepared according to relevant published literature procedures that are used by one skilled in the art. See, e.g., U.S. Patent No. 10,479,797 and U.S. Patent Publication No. 2020-0385395, each of which is incorporated herein in its entirety.

### Compositions and Formulations

Formulations may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tableting lubricants and disintegrants may be used in tablets and capsules for oral administration. Liquid preparations for oral administration may be in the form of solutions, emulsions, aqueous or oily suspensions and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives and flavorings and colorants may be added to the liquid preparations. Parenteral dosage forms may be prepared by dissolving the compound provided herein in a suitable liquid vehicle and filter sterilizing the solution before filling and sealing an appropriate vial or ampule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro et. al*.*)*.*

While it is possible that, for use in the prophylaxis or treatment, a compound provided herein may, in an alternative use, be administered as a raw or pure chemical, it is preferable however to present the compound or active ingredient as a pharmaceutical formulation or composition further comprising a pharmaceutically acceptable carrier.

Pharmaceutical formulations include those suitable for oral, rectal, nasal, topical (including buccal and sub-lingual), vaginal or parenteral (including intramuscular, subcutaneous and intravenous) administration or in a form suitable for administration by inhalation, insufflation or by a transdermal patch. Transdermal patches dispense a drug at a controlled rate by presenting the drug for absorption in an efficient manner with minimal degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds provided herein, together with a conventional adjuvant, carrier, or diluent, may thus be placed into the form of pharmaceutical formulations and unit dosages thereof and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

Compounds provided herein can be used as active ingredients in pharmaceutical compositions, specifically as beta-3 adrenergic receptor modulators. The term "active ingredient", defined in the context of a "pharmaceutical composition"," refers to a component of a pharmaceutical composition that provides the primary pharmacological effect, as opposed to an "inactive ingredient" which would generally be recognized as providing no pharmaceutical benefit.

The dose when using the compounds provided herein can vary within wide limits and as is customary and is known to the physician or other clinician, it is to be tailored to the individual conditions in each individual case. It depends, for example, on the nature and severity of the illness to be treated, on the condition of the patient, on the compound employed or on whether an acute or chronic disease state is treated, or prophylaxis conducted, or on whether further active compounds are administered in addition to the compounds provided herein. Representative doses include, but are not limited to, about 0.001 mg to about 5000 mg, about 0.001 mg to about 2500 mg, about 0.001 mg to about 1000 mg, about 0.001 mg to about 500 mg, about 0.001 mg to about 250 mg, about 0.001 mg to 100 mg, about 0.001 mg to about 50 mg and about 0.001 mg to about 25 mg. Multiple doses may be administered during the day, especially when relatively large amounts are deemed to be needed, for example 2, 3, or 4 doses. Depending on the individual and as deemed appropriate from the healthcare provider it may be necessary to deviate upward or downward from the doses described herein.

The amount of active ingredient required for use in treatment will vary not only with the route of administration, but also the nature of the condition being treated and the age and condition of the patient and will ultimately be at the discretion of the attendant physician or clinician. In general, one skilled in the art understands how to extrapolate *in vivo* data obtained in a model system, typically an animal model, to another, such as a human. In some circumstances, these extrapolations may merely be based on the weight of the animal model in comparison to another, such as a mammal, preferably a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated, or prophylaxis conducted, or on whether further active compounds are administered in addition to the compounds provided herein and as part of a drug combination. The dosage regimen for treating a disease condition with the compounds and/or compositions provided herein is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods provided herein.

The desired dose may conveniently be presented in a single dose or as divided doses administered at appropriate intervals, for example, as two, three, four, or more sub-doses per day. The sub-dose itself may be further divided, *e.g*., into a number of discrete loosely spaced administrations. The daily dose can be divided, especially when relatively large amounts are administered as deemed appropriate, into several, for example two, three, or four-part administrations. If appropriate, depending on individual behavior, it may be necessary to deviate upward or downward from the daily dose indicated.

The compounds provided herein can be administrated in a wide variety of oral and parenteral dosage forms. It will be obvious to those skilled in the art that the dosage forms may comprise, as the active component, a compound provided herein.

For preparing pharmaceutical compositions from the compounds provided herein, the selection of a suitable pharmaceutically acceptable carrier can be either solid, liquid or a mixture of both. Solid form preparations include powders, tablets, pills, capsules, cachets, suppositories and dispersible granules. A solid carrier can be one or more substances which may also act as diluents, flavoring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is admixed with the carrier having the necessary binding capacity in suitable proportions and compacted to the desire shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethyl cellulose, a low melting wax, cocoa butter and the like. The term "preparation" refers to the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool and thereby to solidify.

Formulations suitable for vaginal administration may be presented as pessaries, tampons, creams, gels, pastes, foams, or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds provided herein may thus be formulated for parenteral administration (*e.g*. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, *e.g*. sterile, pyrogen-free water, before use.

Aqueous formulations suitable for oral use can be prepared by dissolving or suspending the active component in water and adding suitable colorants, flavors, stabilizing and thickening agents, as desired.

Aqueous suspensions suitable for oral use can be made by dispersing the finely divided active component in water with viscous material, such as natural or synthetic gums, resins, methylcellulose, sodium carboxymethyl cellulose, or other well-known suspending agents.

Also included are solid form preparations which are intended to be converted, shortly before use, to liquid form preparations for oral administration. Such liquid forms include solutions, suspensions and emulsions. These preparations may contain, in addition to the active component, colorants, flavors, stabilizers, buffers, artificial and natural sweeteners, dispersants, thickeners, solubilizing agents and the like.

For topical administration to the epidermis the compounds provided herein may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds provided herein or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds provided herein as an aerosol can be prepared by processes well known to the person skilled in the art. For their preparation, for example, solutions or dispersions of the compounds provided herein in water, water/alcohol mixtures or suitable saline solutions can be employed using customary additives, for example benzyl alcohol or other suitable preservatives, absorption enhancers for increasing the bioavailability, solubilizers, dispersants and others and, if appropriate, customary propellants, for example include carbon dioxide, CFCs, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, *e.g*., gelatin, or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

Some embodiments include a method of producing a pharmaceutical composition for "combination-therapy" comprising admixing at least one compound according to any of the compound embodiments disclosed herein, together with at least one known pharmaceutical agent and a pharmaceutically acceptable carrier.

It is noted that when the beta-3 adrenergic receptor modulators are utilized as active ingredients in pharmaceutical compositions, these are not intended for use in humans only, but in non-human mammals as well. Recent advances in the area of animal health-care mandate that consideration be given for the use of active agents, such as beta-3 adrenergic receptor modulators, for the treatment of a beta-3 adrenergic receptor-associated disease or disorder in companionship animals (*e.g*., cats, dogs, *etc.*) and in livestock animals (*e.g*., horses, cows, *etc.*) Those of ordinary skill in the art are readily credited with understanding the utility of such compounds in such settings.

### Disorders and Methods of Treatment

The compounds disclosed herein are useful in the treatment or prevention of several diseases, disorders, conditions, and/or indications (which are cumulatively referred to herein as "disorders"). One of skill in the art will recognize that when a disorder, or a method of treatment or prevention, is disclosed herein, such disclosure encompasses second medical uses (*e.g*., a compound for use in the treatment of the disorder, use of a compound for the treatment of the disorder, and use of a compound in the manufacture of a medicament for the treatment of the disorder).

In some embodiments, the compounds disclosed herein are useful for the treatment or prevention of a disorder. In some embodiments, the compounds disclosed herein are useful for the treatment or prevention of a subtype of a disorder. In some embodiments, the compounds disclosed herein are useful for the treatment or prevention of a symptom of a disorder.

Provided herein are methods for treating or preventing a beta-3 adrenergic receptor-mediated disorder. In some embodiments, the compounds disclosed herein are useful for the prevention of a beta-3 adrenergic receptor-mediated disorder. In some embodiments, the compounds disclosed herein are useful for the treatment or prevention of a beta-3 adrenergic receptor-mediated disorder.

One aspect of the present invention relates to methods for treating or preventing a beta-3 adrenergic receptor-mediated disorder in an individual, comprising administering to the individual in need thereof, a therapeutically effective amount of a compound of the present invention; a pharmaceutical product of the present invention; or a pharmaceutical composition of the present invention.

One aspect of the present invention relates to methods for treating or preventing heart failure in an individual, comprising administering to the individual in need thereof, a therapeutically effective amount of a compound of the present invention; a pharmaceutical product of the present invention; or a pharmaceutical composition of the present invention.

One aspect of the present invention relates to methods for treating a hypotensive patient or a borderline hypotensive patient, comprising administering to the patient in need thereof, a therapeutically effective amount of a compound of the present invention; a pharmaceutical product of the present invention; or a pharmaceutical composition of the present invention. One aspect of the present invention relates to uses of a compound of the present invention in the manufacture of a medicament for treating or preventing a beta-3 adrenergic receptor-mediated disorder in an individual.

One aspect of the present invention relates to uses of a compound of the present invention in the manufacture of a medicament for treating or preventing heart failure in an individual.

One aspect of the present invention relates to uses of a compound of the present invention in the manufacture of a medicament for treating a hypotensive patient or a borderline hypotensive patient.

One aspect of the present invention relates to uses of a compound of the present invention in the manufacture of a medicament for treating a normotensive patient.

One aspect of the present invention relates to uses of a compound of the present invention in the manufacture of a medicament for treating a hypertensive patient.

One aspect of the present invention relates to uses of a compound of the present invention in the manufacture of a medicament for treating a patient following myocardial infarction.

One aspect of the present invention relates to compounds of the present invention; a pharmaceutical product of the present invention; or a pharmaceutical composition of the present invention; for use in a method of treatment of the human or animal body by therapy.

One aspect of the present invention relates to compounds of the present invention; pharmaceutical products of the present invention; or pharmaceutical compositions of the present invention; for use in a method for treating or preventing a beta-3 adrenergic receptor-mediated disorder in an individual.

In some embodiments, the beta-3 adrenergic receptor-mediated disorder is selected from the list consisting of: heart failure; reduced cardiac performance in heart failure; mortality, reinfarction, and/or hospitalization in connection with heart failure; acute heart failure; acute decompensated heart failure; congestive heart failure; severe congestive heart failure; organ damage associated with heart failure (*e.g*., kidney damage or failure, heart valve problems, heart rhythm problems, and/or liver damage); heart failure due to left ventricular dysfunction; heart failure with normal ejection fraction; cardiovascular mortality following myocardial infarction; cardiovascular mortality in patients with left ventricular failure or left ventricular dysfunction; a condition following myocardial infarction; left ventricular failure; left ventricular dysfunction; class II heart failure using the New York Heart Association (NYHA) classification system; class III heart failure using the New York Heart Association (NYHA) classification system; class IV heart failure using the New York Heart Association (NYHA) classification system; LVEF < 40% by radionuclide ventriculography; and LVEF ≤35% by echocardiography or ventricular contrast angiography.

In some embodiments, the beta-3 adrenergic receptor-mediated disorder is heart failure.

In some embodiments, the beta-3 adrenergic receptor-mediated disorder is reduced cardiac performance in heart failure.

In some embodiments, the beta-3 adrenergic receptor-mediated disorder is mortality, reinfarction, and/or hospitalization in connection with heart failure.

In some embodiments, the beta-3 adrenergic receptor-mediated disorder is acute heart failure.

In some embodiments, the beta-3 adrenergic receptor-mediated disorder is acute decompensated heart failure.

In some embodiments, the beta-3 adrenergic receptor-mediated disorder is congestive heart failure.

In some embodiments, the beta-3 adrenergic receptor-mediated disorder is severe congestive heart failure.

In some embodiments, the beta-3 adrenergic receptor-mediated disorder is organ damage associated with heart failure (*e.g*., kidney damage or failure, heart valve problems, heart rhythm problems, and/or liver damage).

In some embodiments, the beta-3 adrenergic receptor-mediated disorder is heart failure due to left ventricular dysfunction.

In some embodiments, the beta-3 adrenergic receptor-mediated disorder is heart failure with normal ejection fraction.

In some embodiments, the beta-3 adrenergic receptor-mediated disorder is cardiovascular mortality following myocardial infarction.

In some embodiments, the beta-3 adrenergic receptor-mediated disorder is cardiovascular mortality in patients with left ventricular failure or left ventricular dysfunction.

In some embodiments, the beta-3 adrenergic receptor-mediated disorder is following myocardial infarction.

In some embodiments, the beta-3 adrenergic receptor-mediated disorder is left ventricular failure.

In some embodiments, the beta-3 adrenergic receptor-mediated disorder is left ventricular dysfunction.

Doctors can classify the patient's heart failure according to the severity of their symptoms. The table below describes the most commonly used classification system, the New York Heart Association (NYHA) Functional Classification. It places patients in one of four categories based on how much they are limited during physical activity.

| Class | Patient Symptoms |
|---|---|
| I | No limitation of physical activity. Ordinary physical activity does not cause undue fatigue, palpitation, dyspnea (shortness of breath). |
| II | Slight limitation of physical activity. Comfortable at rest. Ordinary physical activity results in fatigue, palpitation, dyspnea (shortness of breath). |
| III | Marked limitation of physical activity. Comfortable at rest. Less than ordinary activity causes fatigue, palpitation, or dyspnea. |
| IV | Unable to carry on any physical activity without discomfort. Symptoms of heart failure at rest. If any physical activity is undertaken, discomfort increases. |

Accordingly, in some embodiments, the beta-3 adrenergic receptor-mediated disorder is class II heart failure using the New York Heart Association (NYHA) classification system.

In some embodiments, the beta-3 adrenergic receptor-mediated disorder is class III heart failure using the New York Heart Association (NYHA) classification system.

In some embodiments, the beta-3 adrenergic receptor-mediated disorder is class IV heart failure using the New York Heart Association (NYHA) classification system.

In some embodiments, the beta-3 adrenergic receptor-mediated disorder is LVEF < 40% by radionuclide ventriculography.

In some embodiments, the beta-3 adrenergic receptor-mediated disorder is LVEF ≤35% by echocardiography or ventricular contrast angiography.

Other uses of the disclosed receptors and methods will become apparent to those skilled in the art based upon, *inter alia,* a review of this disclosure.

As will be recognized, the steps of the methods of the present invention need not be performed any particular number of times or in any particular sequence. Additional objects, advantages and novel features of this invention will become apparent to those skilled in the art upon examination of the following examples thereof, which are intended to be illustrative and not intended to be limiting.

### EXAMPLES

### Example 1: Stable-Form Screen

The objective of the study was to identify the most stable crystal form for APD418 mesylate under ambient conditions.

### Procedures for Stable-Form Screen

Compound C was slurried in various solvents (Table 1) for 2 weeks at ambient temperature (21°C ± 1°C). Where possible, excess compound was added to the solvent/solvent mixtures and capped in glass high-performance liquid chromatographic (HPLC) vials to ensure that the media had adequate solid material to form a suspension and the suspensions had at least enough solid to recover for PXRD analysis. Slurries were obtained for all samples except for dimethyl sulfoxide that remained a solution. Solubility was determined after 1 day of equilibration and slurries were allowed to stir for 2 weeks. The temperature was monitored and determined to be 21°C ± 1°C for the duration of the study. At the end of 2 weeks, the slurries were removed from stirring and solids were isolated by centrifuge filtration using 0.45-µm nylon filters, allowed to air-dry overnight, and then analyzed by PXRD. Selected samples were also analyzed by TGA and DSC.

**Table 1: Solvents**

| |
|---|
| methyl *t*-butyl ether |
| ethyl acetate |
| methyl ethyl ketone |
| Tetrahydrofuran |
| ethyl alcohol |
| isopropyl alcohol |
| Acetone |
| Acetonitrile |
| methyl alcohol |
| Water |
| water/isopropyl alcohol (1:20) |
| water/acetone (1:20) |
| water/acetonitrile (1:20) |
| dimethyl sulfoxide |

### Procedures for Solubility Analysis

Solubility was determined by gravimetric analysis after ~24 hours of equilibration at ambient temperature (21°C ± 1°C). Samples were separated by centrifuge filtration using an Eppendorf 5417C model centrifuge. Suspension samples were placed into centrifuge filter tubes with 0.45-µm nylon membrane filter and spun at 10000 rpm for 1 minute. Filtrate aliquots of 10 to 20 µL were placed into separate thermogravimetric analyzer pans, previously tared on a TA Instruments Q5000. The filtrates were dried to achieve dry residual solid weights. Solubility was calculated by dividing the dry residual weight by the aliquot volume.

### Procedures for Powder X-Ray Diffraction

X-ray diffraction analysis was performed using a PANalytical. X'Pert PRO MPD powder X-ray diffractometer (EQ0233). Samples were prepared by placing several milligrams of compound onto a sample holder and smoothing flat.

### Procedures for Thermogravimetric Analysis

TGA was performed on a TA Instruments Q5000 (EQ1982).

### Procedures for Differential Scanning Calorimetry

DSC was performed on a TA Instruments DSC Q2000 (EQ1980).

### Procedures for Dynamic Moisture Sorption

Dynamic Moisture Sorption analysis was performed using a TA Instruments Q5000 SA (EQ2418). The sample was prepared for DMS analysis by placing ~5 mg of compound into a tared sample holder.

### Results

Solubility results are provided below. The results showed a number of slurry samples with solubility greater than 8 mM. Compound A has low solubility (≤ 1 mM) in most organic solvents, with slightly improved solubility in acetonitrile, and has high solubility (> 20 mM) in methanol, dimethyl sulfoxide, and aqueous systems.

| **Solvent** | **Solubility (mg/mL)** | **Solubility (mM)** |
|---|---|---|
| methyl t-butyl ether | 0.54 | 0.76 |
| ethyl acetate | 0.63 | 0.88 |
| methyl ethyl ketone | 0.71 | 0.99 |
| Tetrahydrofuran | 0.28 | 0.39 |
| ethyl alcohol | 0.73 | 1.03 |
| isopropyl alcohol | 0.72 | 1.01 |
| Acetone | 0.44 | 0.61 |
| Acetonitrile | 1.66 | 2.31 |
| methyl alcohol | 15.5 | 21.6 |
| Water | 30.5 | 52.6 |
| water/isopropyl alcohol (1:20) | 21.2 | 29.4 |
| water/acetone (1:20) | 33.9 | 47.3 |
| water/acetonitrile (1:20) | 69.5 | 97.0 |
| dimethyl sulfoxide | >437 | >610 |

Compound C was slurried in 13 solvent systems including organic solvents, water, and mixed solvent systems. Greater than 8 mM solubility was obtained in at least 5 slurry systems (Table 4; mostly aqueous or mixed systems) allowing for confidence in the screen. All systems resulted in Form 2 as the final form irrespective of the solvent system (aqueous, part aqueous, or anhydrous). No other new polymorphs were observed. TGA analysis of selected samples indicated a weight loss on the TGA in the range of 0.7 to 1.1% up to 110°C with an additional loss of 0.02 to 0.15% by melting. These results were consistent with hemihydrate stoichiometry that had been later established for this crystal form. The hemihydrate did not convert to an anhydrous form, even after being slurried in 9 different organic solvents with no water added. This result supports the assertion that the compound has a very low critical water activity. Additionally, DSC results showed endotherms (attributed to melting) with onsets in the range of 168 °C to 176°C with no other significant thermal events. Since no other forms were observed, and the only other known crystal form (Form 1) was shown to convert to Form 2 in water, Form 2 is designated as the stable form at ambient conditions.

### SFS Results After 2-Weeks Slurry

| **Solvent** | **Form by PXRD Pattern** | **TGA Weight Loss Initial to 110°C (%wt)** | **TGA Weight Loss 110°C to 175°C (%wt)** | **DSC Melting Onset/Enthalpy of Fusion** |
|---|---|---|---|---|
| methyl *t*-butyl ether | 2 | Not tested | Not tested | Not tested |
| ethyl acetate | 2 | Not tested | Not tested | Not tested |
| methyl ethyl ketone | 2 | Not tested | Not tested | Not tested |
| Tetrahydrofuran | 2 | 0.87 | 0.15 | Not tested |
| ethyl alcohol | 2 | 1.09 | 0.08 | Not tested |
| isopropyl alcohol | 2 | 1.07 | 0.09 | Not tested |
| Acetone | 2 | Not tested | Not tested | Not tested |
| Acetonitrile | 2 | 1.07 | 0.06 | Not tested |
| methyl alcohol | 2 | 1.00 | 0.03 | 175.3°C/67 J/g |
| Water | 2 | 0.95 | 0.07 | 174.0°C/64 J/g |
| water/isopropyl alcohol (1:20) | 2 | 1.02 | 0.08 | 175.6°C/65 J/g |
| water/acetone (1:20) | 2 | 1.02 | 0.05 | 175.7°C/66 J/g |
| water/acetonitrile (1:20) | 2 | 0.72 | 0.02 | 175.0°C/poor baseline |

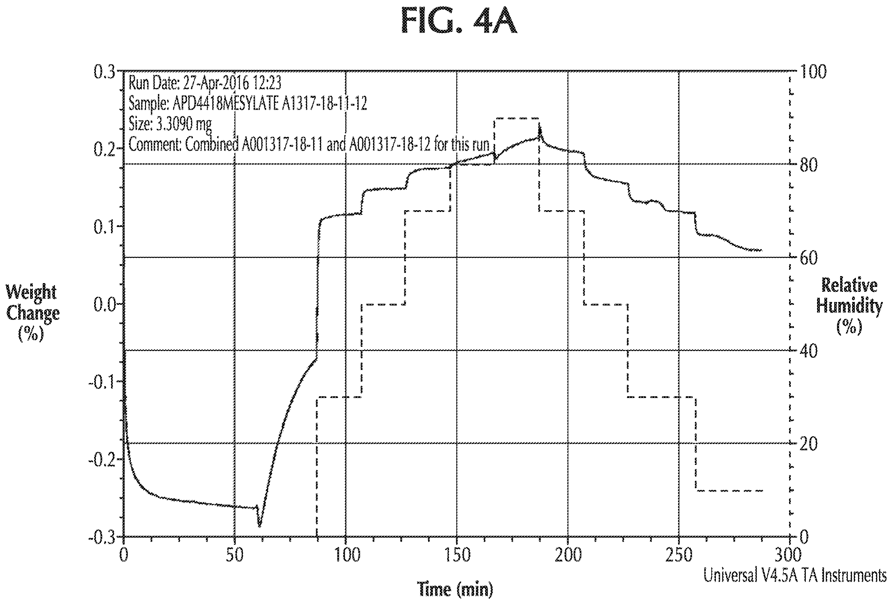

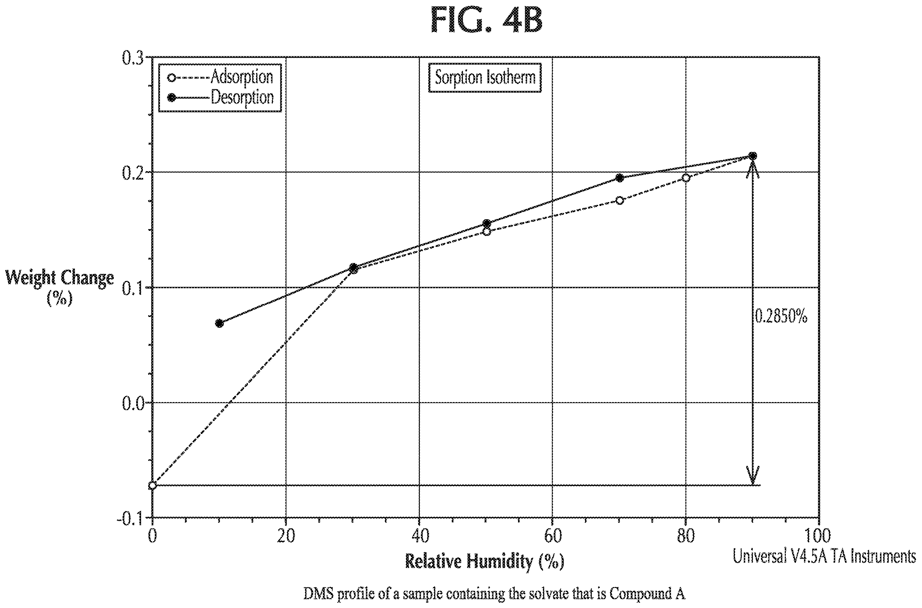

Compound A was shown to maintain its hemihydrate lattice at 25°C and very low humidity/water activity conditions. The representative characterization of this form is shown in Figures 1-4B. The TGA results indicate a ~1% loss of weight that starts at ~30-40°C when exposed to dry gas in the TGA chamber. No other significant weight losses are observed. A corresponding broad and shallow endotherm is seen in the DSC. These events are attributed to the loss of water (hemihydrate form is equivalent ~1.24% weight) when exposed to strong drying conditions. However, this purported loss of water is not seen on the DMS where the total weight change of water is about 0.35% during the drying step, with condition 40°C and ~1% RH until a steady weight was achieved in ~1 hour. The form is considered non-hygroscopic as the DMS showed only a total pick of 0.3% water during the DMS scan from 0 to 90% RH and 25°C. After essentially fully rehydrating, the weight change between 30% RH and 90% RH at 25°C was only ~0.1%.

### Conclusion

Form 1 converts to Form 2 in water. In addition, the stable-form screen indicated that Form 2 does not convert to any other form at ambient temperature. No new PXRD patterns were observed from the screen.

### Example 2: Aqueous Solubility

Solubility of Compound A in aqueous media was measured at various pH values. The conjugate acid ionization constant and intrinsic solubility of the free base were derived by fitting the measured pH-solubility data to the slope-intercept form of the Henderson-Hasselbalch equation for monobasic molecules. The intrinsic solubility of the free base of Compound C (Compound D) was determined to be 0.53 mg/mL and pKa determined to be 7.44. The HCl salt had an aqueous solubility of 5.3 mgA/mL (A = active free base), while the mesylate salt showed aqueous solubility of 28 mgA/mL. The pHmax associated with the mesylate salt was determined to be 5.7.

### Example 3: Single Crystal Structure Determination

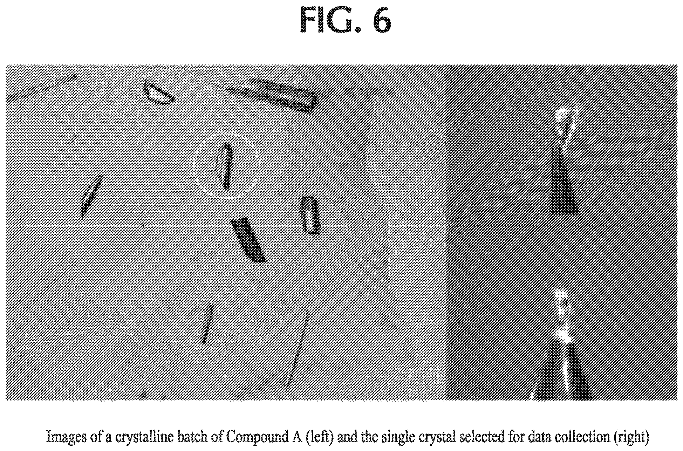

The single crystal X-ray structure of Compound A was determined at 100 K (-173°C) using a crystal obtained by maturation from an acetonitrile and 1.5% H2O solution. See Fig. 6. The crystals are triclinic, space group P1 with the final R₁ = 3.02% for intensity data having [I > 2σ(I)]. In the asymmetric unit, there are 2 molecules of protonated APD418, 2 CH₃SO₃⁻ counter-anions and 1 water molecule, all fully ordered. The unit cell had the following dimensions: *a* = 8.7267(2) Å, *b* = 10.6750(3) Å, *c* = 18.3096(5) Å, α= 75.811, β= 89.605, χ = 80.968. The crystal habit was colorless cut block.

The absolute stereochemistry of the compound has been determined. The stereocenter for the chiral carbon in the 5-membered ring is in the R configuration whereas the stereocenter for the hydroxylated alkyl chiral carbon is in the S configuration. The Flack parameter is -0.008.

The simulated PXRD pattern from the crystal structure model (corresponding to 100 K data collection) is consistent with the experimental diffractogram for the bulk sample with small systematic shifts in peak positions caused by the difference in data collection temperature. This result confirms that the single crystal structure is representative of the bulk material, which was shown to be consistent with Form 2.

Those skilled in the art will recognize that various modifications, additions, substitutions, and variations to the illustrative examples set forth herein can be made without departing from the spirit of the invention and are, therefore, considered within the scope of the invention.

The invention includes the following embodiments:
Embodiment 1: A solvate that is 1-ethyl-3-((*R*)-3-((S)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 2 (Compound A).
Embodiment 2: The solvate of embodiment 1, wherein the solvate exhibits a change in weight of about 0.3% by dynamic moisture-sorption analysis at 25°C and ~0 to 90% relative humidity.
Embodiment 3: The solvate of embodiment 1 or 2, wherein the solvate displays an X-ray powder diffraction pattern comprising peaks, in terms of 2*θ*, at 15.3° ± 0.2°, 19.1° ± 0.2°, 17.8° ± 0.2°, and 18.9° ± 0.2°.
Embodiment 4: The solvate of embodiment 1 or 2, wherein the solvate displays an X-ray powder diffraction pattern comprising peaks, in terms of 2*θ*, at 15.3° ± 0.2°, 19.1° ± 0.2°, 17.8° ± 0.2°, 18.9° ± 0.2°, 26.6° ± 0.2°, 22.9° ± 0.2°, and 8.8° ± 0.2°.
Embodiment 5: The solvate of embodiment 1 or 2, wherein the solvate displays an X-ray powder diffraction pattern comprising peaks, in terms of 2*θ*, at 15.3° ± 0.2°, 19.1° ± 0.2°, 17.794° ± 0.2°, 18.9° ± 0.2°, 26.6° ± 0.2°, 22.9° ± 0.2°, 8.8° ± 0.2°, 12.6° ± 0.2°, 11.4° ± 0.2°, 18.3° ± 0.2°, and 19.9° ± 0.2°.
Embodiment 6: The solvate of embodiment 1 or 2, wherein the solvate displays a powder X-ray diffraction pattern substantially as depicted in Figure 1.
Embodiment 7: The solvate of any one of the preceding embodiments, wherein the solvate displays a differential scanning calorimetry thermogram comprising an endotherm with an extrapolated onset temperature between about 168 °C and about 176 °C.
Embodiment 8: The solvate of any one of the preceding embodiments, wherein the solvate has a differential scanning calorimetry thermogram substantially as depicted in Figure 2.
Embodiment 9: The solvate of any one of the preceding embodiments, wherein the solvate displays a thermogravimetric analysis profile showing about 0.97% weight loss prior to melt.
Embodiment 10: The solvate of any one of the preceding embodiments, wherein the solvate displays a thermogravimetric analysis profile substantially as depicted in Figure 3.
Embodiment 11: The solvate of any one of the preceding embodiments, wherein the solvate displays an aqueous solubility of about 28 mgA/mL.
Embodiment 12: The solvate of any one of the preceding embodiments, wherein the solvate is characterized by a triclinic crystal system, as determined by single crystal X-ray analysis.
Embodiment 13: The solvate of any one of the preceding embodiments, wherein the solvate is characterized by a P1 space group, as determined by single crystal X-ray analysis.
Embodiment 14: The solvate of any one of the preceding embodiments, wherein the solvate is characterized by a unit cell, as determined by single crystal X-ray analysis, of the following dimensions: *a* = 8.7267(2) Å, *b* = 10.6750(3) Å, *c* = 18.3096(5) Å, α= 75.811, β= 89.605, χ = 80.968.
Embodiment 15: The solvate of any one of the preceding embodiments, wherein the solvate is characterized by a colourless cut block habit.
Embodiment 16: A process for making a solvate that is Compound A comprising the steps of: contacting 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate with acetonitrile, and solating the solvate that is Compound A.
Embodiment 17: A process for making a solvate that is Compound A comprising the steps of: contacting 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate Form 1 with water, and isolating the solvate that is Compound A.
Embodiment 18: A solvate that is Compound A prepared by the process of embodiment 16 or 17.
Embodiment 19: A composition comprising the solvate of any one of embodiments 1 to 15 or 18.
Embodiment 20: A pharmaceutical composition comprising the solvate of any one of embodiments 1 to 15 or 18 and a pharmaceutically acceptable carrier.
Embodiment 21: A dosage form comprising the solvate of any one of embodiments 1 to 15 or 18 and a pharmaceutically acceptable carrier.
Embodiment 22: A method for treating or preventing a beta-3 adrenergic receptor-mediated disorder in an individual, comprising administering to said individual in need thereof, a therapeutically effective amount of the solvate of any one of embodiments 1 to 15 or 18; a pharmaceutical composition of embodiment 20; or a dosage form of embodiment 21.
Embodiment 23: A method for treating or preventing a disorder in an individual, comprising administering to said individual in need thereof, a therapeutically effective amount the solvate of any one of embodiments 1 to 15 or 18; a pharmaceutical composition of embodiment 20; or a dosage form of embodiment 21; wherein said disorder is selected from heart failure; cardiac performance in heart failure; mortality, reinfarction, and/or hospitalization in connection with heart failure; acute heart failure; acute decompensated heart failure; congestive heart failure; severe congestive heart failure; organ damage associated with heart failure (e.g., kidney damage or failure, heart valve problems, heart rhythm problems, and/or liver damage); heart failure due to left ventricular dysfunction; heart failure with normal ejection fraction; cardiovascular mortality following myocardial infarction; cardiovascular mortality in patients with left ventricular failure or left ventricular dysfunction; left ventricular failure; left ventricular dysfunction; class II heart failure using the New York Heart Association (NYHA) classification system; class III heart failure using the New York Heart Association (NYHA) classification system; class IV heart failure using the New York Heart Association (NYHA) classification system; LVEF < 40% by radionuclide ventriculography; and LVEF ≤35% by echocardiography or ventricular contrast angiography.
Embodiment 24: A solvate of any one of embodiments 1 to 15 or 18 for use in treating or preventing a beta-3 adrenergic receptor-mediated disorder.
Embodiment 25: A solvate of any one of embodiments 1 to 15 or 18 for use in treating or preventing a disorder that is selected from heart failure; cardiac performance in heart failure; mortality, reinfarction, and/or hospitalization in connection with heart failure; acute heart failure; acute decompensated heart failure; congestive heart failure; severe congestive heart failure; organ damage associated with heart failure (e.g., kidney damage or failure, heart valve problems, heart rhythm problems, and/or liver damage); heart failure due to left ventricular dysfunction; heart failure with normal ejection fraction; cardiovascular mortality following myocardial infarction; cardiovascular mortality in patients with left ventricular failure or left ventricular dysfunction; left ventricular failure; left ventricular dysfunction; class II heart failure using the New York Heart Association (NYHA) classification system; class III heart failure using the New York Heart Association (NYHA) classification system; class IV heart failure using the New York Heart Association (NYHA) classification system; LVEF < 40% by radionuclide ventriculography; and LVEF ≤35% by echocardiography or ventricular contrast angiography.
Embodiment 26: Use of a solvate of any one of embodiments 1 to 15 or 18 in manufacturing a medicament for treating or preventing a beta-3 adrenergic receptor-mediated disorder.
Embodiment 27: Use of a solvate of any one of embodiments 1 to 15 or 18 in manufacturing a medicament for in treating or preventing a disorder that is selected from heart failure; cardiac performance in heart failure; mortality, reinfarction, and/or hospitalization in connection with heart failure; acute heart failure; acute decompensated heart failure; congestive heart failure; severe congestive heart failure; organ damage associated with heart failure (e.g., kidney damage or failure, heart valve problems, heart rhythm problems, and/or liver damage); heart failure due to left ventricular dysfunction; heart failure with normal ejection fraction; cardiovascular mortality following myocardial infarction; cardiovascular mortality in patients with left ventricular failure or left ventricular dysfunction; left ventricular failure; left ventricular dysfunction; class II heart failure using the New York Heart Association (NYHA) classification system; class III heart failure using the New York Heart Association (NYHA) classification system; class IV heart failure using the New York Heart Association (NYHA) classification system; LVEF < 40% by radionuclide ventriculography; and LVEF ≤35% by echocardiography or ventricular contrast angiography.

## Claims

1. A solvate that is 1-ethyl-3-((*R*)-3-((*S*)-2-hydroxy-3-(3-(methylsulfonyl)phenoxy)propylamino)-1-oxa-8-azaspiro[4.5]decan-8-ylsulfonyl)quinolin-4(1*H*)-one mesylate hemihydrate Form 2 (Compound A).

2. The solvate of claim 1, wherein the solvate exhibits a change in weight of about 0.3% by dynamic moisture-sorption analysis at 25°C and ~0 to 90% relative humidity.

3. The solvate of claim 1 or 2, wherein the solvate displays an X-ray powder diffraction pattern comprising peaks, in terms of 2*θ*, at 15.3° ± 0.2°, 19.1° ± 0.2°, 17.8° ± 0.2°, and 18.9° ± 0.2°.

4. The solvate of claim 1 or 2, wherein the solvate displays an X-ray powder diffraction pattern comprising peaks, in terms of 2*θ*, at 15.3° ± 0.2°, 19.1° ± 0.2°, 17.8° ± 0.2°, 18.9° ± 0.2°, 26.6° ± 0.2°, 22.9° ± 0.2°, and 8.8° ± 0.2°.

5. The solvate of claim 1 or 2, wherein the solvate displays an X-ray powder diffraction pattern comprising peaks, in terms of 2*θ*, at 15.3° ± 0.2°, 19.1° ± 0.2°, 17.794° ± 0.2°, 18.9° ± 0.2°, 26.6° ± 0.2°, 22.9° ± 0.2°, 8.8° ± 0.2°, 12.6° ± 0.2°, 11.4° ± 0.2°, 18.3° ± 0.2°, and 19.9° ± 0.2°.

6. The solvate of claim 1 or 2, wherein the solvate displays a powder X-ray diffraction pattern substantially as depicted in Figure 1.

7. The solvate of any one of the preceding claims, wherein the solvate displays a differential scanning calorimetry thermogram comprising an endotherm with an extrapolated onset temperature between about 168 °C and about 176 °C.

8. The solvate of any one of the preceding claims, wherein the solvate has a differential scanning calorimetry thermogram substantially as depicted in Figure 2.

9. The solvate of any one of the preceding claims, wherein the solvate displays a thermogravimetric analysis profile showing about 0.97% weight loss prior to melt.

10. The solvate of any one of the preceding claims, wherein the solvate displays a thermogravimetric analysis profile substantially as depicted in Figure 3.

11. The solvate of any one of the preceding claims, wherein the solvate displays an aqueous solubility of about 28 mgA/mL.

12. The solvate of any one of the preceding claims, wherein the solvate is **characterized by** a triclinic crystal system, as determined by single crystal X-ray analysis.

13. The solvate of any one of the preceding claims, wherein the solvate is **characterized by** a P1 space group, as determined by single crystal X-ray analysis.

14. The solvate of any one of the preceding claims, wherein the solvate is **characterized by** a unit cell, as determined by single crystal X-ray analysis, of the following dimensions: a = 8.7267(2) Å, *b =* 10.6750(3) Å, *c* = 18.3096(5) Å, α= 75.811, β= 89.605, χ = 80.968.

15. The solvate of any one of the preceding claims, wherein the solvate is **characterized by** a colourless cut block habit.
